Europäisches Patentamt

European Patent Office

Office européen des brevets

(19)

(11) Veröffentlichungsnummer: **0 150 428**
**B1**

(12) # EUROPÄISCHE PATENTSCHRIFT

(45) Veröffentlichungstag der Patentschrift: 06.06.90

(51) Int. Cl.⁵: **A 61 L 15/24, A 61 K 9/70**

(21) Anmeldenummer: 84115665.6

(22) Anmeldetag: 18.12.84

(54) Wirkstoffabgabesysteme.

(30) Priorität: 28.12.83 DE 3347278

(43) Veröffentlichungstag der Anmeldung:
07.08.85 Patentblatt 85/32

(45) Bekanntmachung des Hinweises auf die
Patenterteilung:
06.06.90 Patentblatt 90/23

(84) Benannte Vertragsstaaten:
AT BE CH DE FR GB IT LI NL SE

(56) Entgegenhaltungen:
EP-A-0 144 486      GB-A-2 073 588
EP-A-0 148 391      GB-A-2 095 108
FR-A-2 497 457      US-A-3 742 951
GB-A-2 021 950

PATENTS ABSTRACTS OF JAPAN, Band 7, Nr.
155 (C-175)1300r, 7. Juli 1983; & JP - A - 58 67
617 (NITTO DEKI KOGYO K.K.) 22-04-1983

Die Akte enthält technische Angaben, die nach
dem Eingang der Anmeldung eingereicht
wurden und die nicht in dieser Patentschrift
enthalten sind.

(73) Patentinhaber: BAYER AG
D-5090 Leverkusen 1 Bayerwerk (DE)

(72) Erfinder: Von Bittera, Miklos
Max-Scheler-Strasse 7
D-5090 Leverkusen 3 (DE)
Erfinder: Meyer, Rolf-Volker, Dr.
Buchheimer Strasse 23
D-4150 Krefeld (DE)

# EP  0 150 428  B1

**Beschreibung**

Die Erfindung betrifft ein untenstehend im einzelnen gekennzeichnetes medizinisches Pflaster zur transdermalen Verabreichung von Etofenamat über einen längeren Zeitraum.

In der US—A—4 031 894 werden medizinische Pflaster beschrieben, die ein Reservoir aus einer Mischung von Polyisobutylenen mit sehr verschiedenen Molgewichten, nämlich M.G. 35 000 bis 50 000 und 1 000 000 bis 1 500 000 und Mineralölen besitzen.

Diese Pflaster sind nur für Wirkstoffe, die in sehr geringen Dosen appliziert werden, geeignet. In der US—PS wird Scopolamin genannt.

In der GB—A—2 095 108 werden medizinische Pflaster zur Applikation von Arzneimitteln offenbart. Unter anderem werden in einer allgemeinan Aufzählung auch Antiphlogistika, z.B. Indomethacin, genannt. Als Polymerbasis werden allgemein Kautschuke mit bestimmten Gasübergangstemperaturen eingesetzt, die einen guten Tragkomfort gewährleisten, die jedoch bezüglich der Freisetzungsrate von Etofenamat zu wünschen übrig lassen.

Gegenstand der US—A—3 742 951 sind Systeme zur Freisetzung von mikroverkapselten Wirkstoffen, insbesondere Nitroglycerin, die in eine Polymerbasis aus Polyethylen und Acrylaten eingebettet sind. Die Freisetzungsraten von Etofenamat aus solchen Systemen sind unbefriedigend.

Die britische Offenlegungsschrift GB—A—2 073 588 betrifft eine Haftklebeschicht von Pflastern zur Applikation von Nitroglyzerin. Als Polymerbasis werden Naturkautschuk oder Naturkautschuk-Verschnitte eingesetzt. Eine Beimischung z.B. von niedrig-molekularem Polyisobutylen (20%) dient hier zur Verbesserung der Kautschukeingenschaften bezüglich Flexibilität und Gasundurchlässigkeit. Naturkautschuke dieser Art sind für Etofenamat nicht geeignet.

In der GB—A—2 045 618 werden Pflaster auf Basis von Styrol/Isopren/Styrol-Blockcopolymeren zur Applikation von Antiphlogistika beschrieben. Als Beispiel wird u.a. Etofenamat genannt. Die Freisetzungsrate dieser Pflaster ist jedoch unbefriedigend.

Gegenstand der GB—A—2 021 950 sind nitroglycerinhaltige Salze, die auf Polymerbandagen aufgetragen werden. Diese Art von Wirkstoffabgabesystemen sind für Etofenamat nicht geeignet.

In der EP—A—148 391 werden etofenamathaltige Pflaster auf Basis von Polyisobutylen-Polymeren mit bestimmten Molekulargewichtsverteilungen beschrieben.

In der EP—A—144 486 werden Pflaster, bestehend aus einem Wirkstoffreservoir und einer Haftklebschicht offenbart. Das Wirkstoffreservoir besteht aus mehreren Schichten auf Basis von Polyisobutylen, wobei die einzelnen Schichten steigende Wirkstoffkonzentration mit zunehmenden Abstand von der Haut haben. Als geeignete Wirkstoffe werden in einer Aufzählung allgemein auch Antirheumatika genannt.

Die FR—A—2 497 457 offenbart Pflaster zur Applikation von Nitraten (ISDN, PETN), die bei einer befriedigenden Freisetzungsrate für die Nitrate einen guten Tragkomfort gewährleisten. Dies wird erreicht durch Verwendung von Polymeren, die eine bestimmte Glasübergangstemperatur haben.

Diese Bedingung wird beispielsweise von Acrylpolymeren, Isopen-Kautschuken sowie von PIB-Gemischen mit niederem und hohem Molekulargewicht erfüllt. Die Freisetzungsrate solcher Polymeren sind jedoch für Etofenamat sehr unbefriedigend.

Bekannte Wirkstoffabgabesysteme, wie z.B. Gele, Salben, bekannte Pflaster u.ä. erlauben nur eine begrenzte Wirkstoffresorption durch die Haut. Die Resorption hängt von der Grundlage und den Wirkstoffeingenschaften ab.

Es ist daher eine Aufgabe der vorliegenden Erfindung, medizinische Pflastern zu entwickeln, mit deren Hilfe über einen längeren Zeitraum geregelte, größere therapeutisch wirksame Mengen von Etofenamat über die Haut verabreicht werden können. Diese Pflaster sollen mit der Haut verträglich sein und mit ihrer Hilfe soll es möglich sein, hohe therapeutisch wirksame Dosen des Wirkstoffs Etofenamat zu verabreichen.

Überraschenderweise wurde nun gefunden, daß man entsprechende medizinische Pflaster mit deutlich erhöhten Abgaberaten von Etofenamat erhält, wenn man als Polymerkomponente spezielle gegebenenfalls mit Styrol modifizierte Dienkautschuke einsetzt.

Die Erfindung betrifft ein medizinisches Pflaster zur transdermalen Verabreichung von Antiphlogistika, bestehend aus einer Deckschicht, einer abziehbaren Schutzschicht und einer den Wirkstoff, Schleppmittel und Harze enthaltenden, einlagigen Reservoirschicht auf Basis von Polymeren dadurch gekennzeichnet, daß die Reservoirschicht als Polymere Polyisobutylene mit einer Molmassen-Verteilung $M_w/M_n$ von 1,5 bis 3,5 und eine mittlere Viskosität/Molmasse von 30 000 bis 4 000 000 g/ol enthält und 1 bis 30 Gew.-% des Wirkstoffs Etofenamats enthält.

Die Wirkstoffreservoirschicht besteht bevorzugt aus 30 bis 60 Gew.-% Polymerkomponente, 30 bis 60 Gew.-% Schleppmittel und 2 bis 40 Gew.-% eines Harzes, wobei die Summe der drei Komponenten 100 Gew.-% beträgt.

Die Butadienkautschuke sind bekannte Produkte, die auf verschiedenen, dem Fachmann bekannten Wegen hergestellt werden können, wobei je nach Wahl des Metallkatalysators die Natur de Doppelbindungen im Polymer breit variierbar ist (s. z.B. Ullmans Enzyklopädie d. technischen Chemie, 4, Aufl. Bd. 13, S. 602 bis 611, Verlag Chemie, Weinheim/New York 1977).

Bevorzugt werden Butadienkautschuke mit über 80% cis-1,4-Verknüpfung eingesetzt.

Auch die mit Stytol modifizierten Butadienkautschuke sind bekanntze Produkte, z.B, als "Styrol-

2

Butadien-Kautschuk", die nach bekannten Verfahren so hergestellt werden können, daß der Styrol-Anteil nicht nur statistisch, sondern auch teilweise bzw. überwiegend als Blockstruktur in dem Butadienkautschuk eingebaut ist. Die erfindungsgemäß verwendbaren Kautschuke weisen Gewichtsmittel der Molmassen $M_w$ von 20 000 bis 2 000 000, vorzugsweise von 20 000 bis 500 000 g/mol auf.

Ganz besonders bevorzugt werden Polybutadien-Kautschuke mit Mooney-Viskositäten von 30 bis 60 (ML 1+4 bei 100°C) eingesetzt.

Unter Schleppmittel im Sinne der vorliegenden Erfindung werden Öle, Fettsäureester, Triglyceride, Alkohole und/oder Fettsäuren verstanden.

Unter Ölen im Sinne der vorliegenden Erfindung werden hochsiedende, aliphatische, araliphatische und/oder aromatische Kohlenwasserstoffe verstanden, vorzugsweise Paraffinöl, Purcellinöl, Perhydrosqualen und Lösungen von mikrokristallinen Wachsen in den Ölen, Mineralöle, bevorzugt Öle, deren Siedebereich zwischen 150°C und 400°C liegt; ferner ungesättigte Kohlenwasserstoffe mit mindestens 16 C-Atomen wie z.B. Oligomere von Monoolefinen wie Tetraisobutylen, Pentaisobutylen, Hexaisobutylen oder auch flüssige Polymerisate aus Dien(Monoen)-(Co)-Polymerisaten. Beispiele für flüssige Polymerisate aus konjugierten Dienen sind solche aus Butadien, Isopren, 1,3-Pentadien, 2,3-Dimethylbutadien, Copolymerisate verschiedener Diene sowie auch flüssige Copolymerisate aus einem konjugierten Diolefin und geringen Mengen von Monoolefinen wie z.B. Buten-1, Isobuten, Hexen-1, Octen-1, Styrol mit MG von 400 bis 6000, vorzugsweise 800 bis 3000 sowie Iodzahlen von 200 bis 500 un Viskositäten von 100—10.000 cP bei 50°C.

Besonders bevorzugt sind flüssige Polybutadien-Polymerisate, die zu mindestens 90% 1,4-verknüpft sind, deren Anteil an cis-Doppelbindungen mehr als 60% beträgt und deren Molmassen 1000—4000 betragen.

Unter Ölen werden auch Silikonöle verschiedener Viskosität, vorzugsweise mit mittleren Molgewichten von 312 bis 15.000, besonders bevorzugt Polydimethylsiloxane, verstanden.

Unter Fettsäureestern werden solche verstanden, die mindestens 12 C-Atome, vorzugsweise 15 bis 46 C-Atome, besonders bevorzugt 16 bis 36 C-Atome enthalten. Insbesondere werden darunter verstanden: Ethylstearat, Laurinsäurehexylester, Dipropylen-glykolpelargonat, Palmitinsäurecetlyester, Isopropylmyristat, Isopropylpalmitat, Capryl/Caprinsäureester von gesättigten Fettalkoholen der Kettenlänge $C_{12}$—$C_{18}$, Isopropylstearat, Ölsäureoleylester, Ölsäuredecylester, Ethyloleat, künstliches Entenbürzeldrüsenfett, und zwar jeweils einzeln oder im Gemisch.

Unter Triglyceriden werden reine oder gemischte Ester des Glycerins mit Fettsäuren der Kettenlänge $C_8$—$C_{18}$ verstanden, vorzugsweise Capryl- und/oder Caprinsäuretriglyceride.

Unter Fettsäuren werden gesättigte oder ungesättigte Fettsäuren, vorzugsweise solche mit 12—24 C-Atomen, einzeln oder im Gemisch miteinander, besonders bevorzugt Ölsäure, verstanden.

Unter Ölen im Sinne der Erfindung werden ferner verstanden:

Süßmandelöl, Avocadoöl, Sesamöl, Rizinusöl, Olivenöl, Traubenkernöl, Nelkenöl, Erdnußöl, Maisöl, Haselnußöl, Jojobaöl, Carthamaöl und Weizenkeimöl, jeweils einzeln oder im Gemisch.

Unter Harzen im Sinne der vorliegenden Erfindung werden Kolophonium, dehydriertes Kolophonium, Glycerinester von dehydriertem Kolophonium, Glycerinester von Kolophonium-gummi, hydriertes Kolophonium, Glycerinester von hydrierten Kolophonium, Pentaerythritester von hydriertem Kolophonium, Methylester von hydriertem Kolophonium, polymerisiertes Kolophonium, Glycerinester von polymerisiertem Kolophonium, Terpenharze, Cumaron/Inden-Harze, hydrierte Petroleumharze, mit Maleinsäureanhydrid modifiziertes Kolophonium und Kolophoniumderivate, $C_5$-Petroleumharze und Halbester von Styrol/Maleinsäure-Co-polymeren einzeln oder im Gemisch miteinander verstanden. Besonders bevorzugt werden Polyterpenharze aus Alpha- bzw. Beta-Pinen oder modifizierte Glycerinester der Kolophoniums. Diese Harze können je nach den erforderlichen Eigenschaften hinsichtlich der Klebrigkeit und der Haftfestigkeit auf dem Teil, an dem das resultierende Pflaster angebracht werden soll, entweder allein oder in Kombination miteinander verwendet werden.

Das Etofenamat kann in einer Menge von 1—30 Gew.-%, vorzugsweise 2—20 Gew.-% in die Reservoirschicht eingearbeitet werden. Die angegebenen Gew.-% beziehen sich auf Gesamtreservoir.

Dem Etofenamat können zusätzlich noch weitere Wirksubstanzen zugesetzt werden oder auch kühlende oder duftabgebende Substanzen, vorzugsweise Methylsalicylat, Glycolsalicylat, Salicylsäure, Menthol, Pfefferminzöl, Kampfer, Thymol, Acrinol, Scopolaextrakt, Chlorpeniraminmaleat, Benzlynicotinat, Capsicumextrakt, Nonylvanillylamid, Capsaicin.

Erforderlichenfalls können die erfindungsgemäßen Pflaster mit Additiven und Füllstoffen, z.B. Alterungsschutzmitteln, Antioxidantien und Verstärkungsfüllstoffen versetzt werden, soweit die gelartigen Eigenschaften nicht zerstört werden.

Bekannte Wirkstoffabgabesysteme, wie z.B. Gele, Salbengrundlagen, Pflaster geben ca. 0,5—5 mg Wirkstoff in 4 Stunden frei. Das oben beschriebene erfindungsgemäße medizinische Pflaster dagegen setzt in 4 Stunden bis zu 18 mg Etofenamat frei mit einer signifikant größeren Bioverfügbarkeit. Die erfindungsgemäßen Systeme können durch Änderung des Polymeranteils, des Schleppmittels bzw. des Harzes bezüglich ihrer Wirkstoffabgabegeschwindigkeit nahezu beliebig eingestellt werden.

Die Herstellung des etofenamathaltigen Reservoirs und des darauf basierenden Pflasters kann z.B. wie folgt durchgeführt werden: Die Pflastergrundstoffe (Polymer, Harz und Schleppmittel) werden in ein geeignetes Lösegefäß eingebracht und unter Rühren in Benzin gelöst. Es resultiert eine klare bis leicht getrübte Lösung 1. Das Etofenamat wird ebenfalls in geeigneten Lösungsmittel gelöst und der Polymerlösung 1 zugesetzt.

Die so erhaltene etofenamathaltige Lösung 2 wird gleichmäßig auf silikonisiertes Papier aufgebracht und zu einem Film ausgezogen. Das beschichtete Papier mit der Pflastergrundlage wird 24 Stunden an der Luft getrocknet und dann 1 Stunde im Umlufttrockenschrank bei 40°C aufbewahrt.

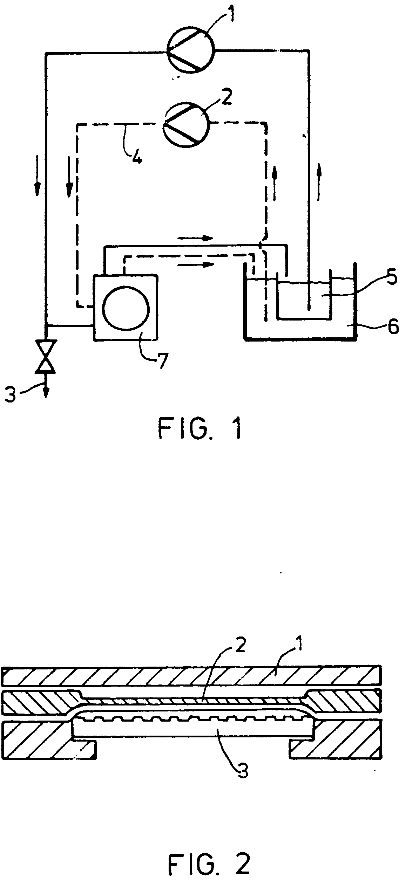

Die Bestimmungen der Wirkstoffabgaberaten erfolgten in einem Resorptionsmodell, das im folgenden und im experimentellen Teil genauer beschrieben ist. Fig. 1 zeigt ein Resorptionsmodell. Fig. 2 zeigt eine Resorptionszelle.

In Fig. 1 bedeutet 1 eine Schlauchpumpe für den Akzeptor, 2 eine Schlauchpumpe zum Temperieren, 3 die Probeentnahme, 4 den Kreislauf für die Temperierflüssigkeit, 5 das Akzeptormedium, 6 das Temperiergefäß und 7 die Resorptionszelle mit Membran.

In Fig. 2 bedeutet 1 eine undurchsichtiges Zellenmaterial, 2 eine Membrane und 3 ein Sichtfenster aus Glas, das zugleich eine Riffelplatte für das Akzeptormedium ist.

In-Vitro Freisetzungsprüfung der erfindungsgemäßen Pflaster

Alle Pflaster wurden auf die gleiche Weise mit 10% Etofenamat hergestellt aus

Polymer
Schleppmittel
Harz

gegebenenfalls Lösungsmittel (Benzin, Hexan oder Hexan Toluol-Mischung). Die jeweils verwendeten Mengenverhältnisse sind bei den Rezepturbeschreibungen aufgeführt.

Hierzu wurden alle Komponenten gelöst oder suspendiert. Als Lösungsmittel für den Wirkstoff wurden überwiegend Aceton und/oder Ethanol verwendet.

Diese Lösungen bzw. Suspensionen wurden zu dünnen Folien einer Dicke von 50—150 µm verarbeitet.

Versuchsparameter:

| | |
|---|---|
| Akzeptormedium: | Mischung aus Wasser, Ethanol, PVP, Sorbitanfettsäureester |
| Volumen Akezptormedium: | 200 ml |
| Temperatur Akzeptormedium: | 35—36°C |
| Pumpleistung: | 16 ml/min. (Gerätekonstante) |
| Membran: | als Membran wurde die in Beispiel 3 der DE—OS 3 312 735 beschriebene Folie eingesetzt |
| Resorptionsfläche: | 33,18 cm² (Zellenkonstante) |

Das Akzeptormedium wurde in einem Vorratsgefäß temperiert und über Schläuche in die Resorptionszelle umgepumpt. Die Probenentnahme erfolgte zwischen der Pumpe und den Resorptionszellen. Die Probenziehung erfolgte in festgelegten Zeitabständen. Es wurden je 6 ml Probe entnommen und spektralphotometrisch vermessen. Ein Ersatz der Akzeptorflüssigkeit erfolgte nicht, da dies eine Verdünnung der Restmenge bedeuten würde.

Berechnung der Ergebnisse

Zunächst wurde eine Eichkurve des Etofenamats aufgenommen, mit deren Hilfe dann aus den für die Einzelproben gemessenen Extinktionswerten die Wirkstoffkonzentration (mg oder %) in den Einzelproben ermittelt wurde. Die Extinktionen wurden UV-spektroskopisch gemessen.

Zur Berechnung der "relativen Resorption" (Anteil "resorbierter" Wirkstoff am Gesamtgehalt des Pflasters in %) is die Kenntnis der eingesetzten Wirkstoffmenge pro Pflasterfläche (33,18 cm²) nötig. Diese ist aus der Herstellung des Pflasters bekannt.

Aus den für die einzelnen Proben gemessenen Extinktionswerden wurde mit Hilfe einer Eichgeraden bzw. dem daraus ermittelten Faktor die Konzentration an Wirkstoff in der Probe ermittelt.

Die Berechnung erfolgte nach den folgenden Formeln

$$M_i(t) = V_t \cdot C_i + M_F(t) \ [mg]$$

$$M_F(t) = \Sigma \sum_{i=0}^{i=n-1} (V_D \cdot C_i) \ [mg]$$

$M_i(t)$: freigesetzte Arzneistoffmenge bis zur Zeit t [mg]

$V_t$: Volumen des Akzeptors zur Zeit t [ml]

$C_i$: Wirkstoffkonzentration in der betreffenden Probe [mg/ml]

$M_F(t)$: entnommene Wirkstoffmenge biz zur Zeit t [mg]

$V_D$: Probevolumen [ml]

n: Anzahl der Proben bis zur Zeit t

t: Versuchsdauer

Herstellungsbeschreibung

Die erfindungsgemäßen medizinischen Pflaster wurden folgendermaßen hergestellt: Das Gemisch aus Polymer, Haz und Schleppmittel wurde in einem z-Kneter bei einer Temperatur von 120 bis 150°C vorgeknetet. Wenn die Masse eine homogene Schmelze darstellt wurde unter Stickstoffbegasung Etofenamat homogen eingearbeitet. Die wirkstoffhaltige Schmelze wurde auf die Trägerfolie aufgetragen (Kneter).

Die erfindungsgemäßen medizinischen Pflaster wurden in einem Lösungsmittelgemisch aufgelöst, auf die Trägerfolie aufgetragen und anschließend getrocknet (Lösung).

Die Erfindung betrifft auch ein Verfahre zur Herstellung der erfindungsgemäßen medizinischen Pflaster das dadurch gekennzeichnet ist, daß man Polybutadien-Kautschuke mit mindestens 30% cis-1,4-Verknüpfung und Mooney-Viskositäten von 20 bis 100 (ML 1+4 bei 100°C), die bis zu 50 Gew.-% entweder statistisch oder in Blöcken (Styrol) eingebaut enthalten können, ein oder mehrere Schleppmittel und ein oder mehrere Harze in einem Lösungsmittel löst, dann 1 bis 30 Gew.-% Etofenamat, bezogen auf das Gemisch aus Polymerkomponente, Schleppmittel und Harz, ebenfalls in einem Lösungsmittel zur Lösung bringt, diese Lösungen vereinigt und die vereinigten Lösungen gleichmäßig auf eine für die Wirkstoffe im wesentlichen undurchlässige Folie (Deckschicht) aufträgt und zu einem Film auszieht, die beschichtete Folie (Deckschicht) zunächst bei Raumtemperatur und anschließend bei Temperaturen bis zu 50°C troknet und gegebenenfalls die getrocknete Folie (Deckschicht) auf der beschichteten Seite mit einer für den Wirkstoff im wesentlichen undurchlässigen abziehbaren Schutzschicht verseiht.

## Beispielserie A

Standard (Vergleich zum Stand der Technik gemäß DE—A—30 07 368)

Bei dieser Versuchsreihe wurde ein Styrol/Isopren/Styrol-Tr-Blockcopolymerisat ("Cariflex TR 1107) von Shell Chem. Co.) als Polymer, Paraffin dünnflüssig als Schleppmittel un Polyterpenharz aus β-Pinen als klebrigmachendes Harz eingesetzt.

Das 10% etofenamathaltige Styrol/Isopren/Styrol-TR-Blockcopolymer-Pflaster wurde in allen weiteren Versuchen als Bezugsstandard verwendet.

Die genaue Zusammensetzung der Pflastergrundlage ist in Tabelle 1 angegeben. Die Herstellung erfolgte wie vorstehend beschrieben. Die Freisetzungsraten sind in Tabelle 2 beschrieben.

## TABELLE 1
### Zusammensetzung der Standard-Formulierung

| | |
|---|---|
| Styrol/Isopren/Styrol/TR-Blockcopolymerisat | 36,0 g |
| Paraffin dünnflüssig | 45,0 g |
| Polyterpenharz aus β-Pinen | 9,0 g |
| Etofenamat | 10,0 g |

## TABELLE 2
### Freisetzung der Standardversuchsserie in Abhängigkeit von der Zeit

| | freigesetzte Menge Etofenamat in mg/h | | | | | | % | Einwaage an Etofenamat in mg |
|---|---|---|---|---|---|---|---|---|
| | 0,5 | 1 | 1,5 | 2 | 3 | 4 | | |
| Standard 10% | 1,44 | 2,16 | 2,70 | 3,24 | 4,63 | 4,81 | 21,20 | 22,77 |

Beispielserie B

Bei dieser Versuchsreihe wurde die Zusammensetzung der Polymeren variiert. Die genaue Bezeichnung ist in Tabelle 3 angegeben. Die Herstellung erfolgte wie vorstehend beschrieben. Alle in Tabelle 3 aufgeführten Polymere wurden unter Konstanthalten des Paraffinöl- bzw. Harzanteils in der Menge nach folgenden Schema variiert:

|  | A1 | A2 |
|---|---|---|
| Polymer | 36,0 g | 45,0 g |
| Paraffin dünnflüssig | 45,0 g | 37,5 g |
| Polyterpenharz aus β-Pinen | 9,0 g | 7,5 g |
| Etofenamat | 10,0 g | 10,0 g |

Die Freisetzungsraten sind in Tabelle 4 beschrieben.

TABELLE 3

Beispielserie B: Beschreibung der eingesetzten Polymeren

| Nr.[1] | Beschreibung |
|---|---|
| 01 × | Styrol-Butadien-Kautschuk (Lösungs-SBR) mit 18% Styrol, Mooney-Viskosität ML(1+4) 100°C:35 |
| 02 × | Styrol-Butadien-Blockcopolymer, hergestellt mit Li-Katalysator, Styrolgehalt 30%, davon 22% als Block, Mw ca. 180.000 g/mol |
| 03 × | Polybutadien-Kautschuk, 93% cis -1,4-Gehalt, 4% 1,2-Gehalt, hergestellt mit Ti-Katalysator, Mooney Viskosität ML(1+4)100°C:35 |
| 04 × | Polybutadien-Kautschuk, 38% cis -1,4-Gehalt, 10% 1,2-Gehalt, hergestellt mit Li-Katalysator, Mooney Viskosität ML(1+4)100°C:35 |
| 05 × | Polybutadien-Kautschuk, 38% cis -1,4-Gehalt, 10% 1,2-Gehalt, hergestellet mit Li-Katalysator, Mooney-Viskosität ML(1+4)100°C:55 |

[1] die beiden ersten Ziffern bezeichnen das Polymer, anstelle von × tritt die Bezeichnung der Formulierung (s. Seite 25; A 1 und A 2)

TABELLE 4

Beispielserie B: Freisetzung in Abhängigkeit von der Zeit

| Nr. | freigesetztes Etofenamat (mg) in h | | | | | | % | Etofenamat Einwaage in mg |
|---|---|---|---|---|---|---|---|---|
|  | 0,5 | 1 | 1,5 | 2 | 3 | 4 | | |
| Standard | 1,44 | 2,16 | 2,70 | 3,24 | 4,63 | 4,81 | 21,20 | 22,77 |
| 01A1 | 3,37 | 5,79 | 8,14 | 9,62 | 12,05 | 13,87 | 54,97 | 25,23 |
| 01A2 | 2,91 | 5,23 | 7,05 | 8,45 | 11,50 | 13,93 | 44,00 | 31,94 |
| 02A1 | 2,50 | 4,43 | 6,25 | 7,92 | 10,79 | 13,09 | 51,66 | 25,34 |
| 02A2 | 2,65 | 4,33 | 6,11 | 7,55 | 10,42 | 12,50 | 51,74 | 24,16 |
| 05A2 | 3,01 | 5,04 | 6,81 | 8,67 | 11,23 | 13,22 | 40,74 | 32,45 |
| 03A1 | 3,11 | 6,67 | 8,83 | 10,41 | 13,86 | 15,81 | 75,56 | 20,03 |
| 03A2 | 2,65 | 4,43 | 6,16 | 7,46 | 10,11 | 12,23 | 37,47 | 32,64 |
| 04A1 | 3,72 | 6,25 | 8,40 | 9,75 | 12,94 | 14,84 | 63,73 | 23,29 |
| 04A2 | 2,14 | 3,62 | 5,54 | 7,03 | 10,13 | 12,34 | 48,02 | 25,69 |
| 05A1 | 2,70 | 4,03 | 5,25 | 6,51 | 8,62 | 10,35 | 33,41 | 30,98 |

Beispielserie C;

Variation der flüssigen Komponente

Die flüssige Komponente der aus Beispielserie B ausgewählten Formulierungen wurde in der Zusammensetzung nach folgendem Schema, unter Beibehaltung der übrigen Rezeptur, geändert.

|  | B1 | B2 |
|---|---|---|
| Polymer | wie in Beispielserie B | |
| flüssige Komponente | Polybutadienöl M.G. 1500 | Polybutadienöl M.G. 1500 +<br>Paraffin dünnflüssig 1:1 |
| Harz | wie in Beispielserie B | |
| Etofenamat | 10% | 10% |

Die genaue Zusammensetzung der Pflastergrundlage sind in Tabelle 5, die Freisetzungsraten in Tabelle 6 angegeben.

TABELLE 5

Beispielserie C: Variation der flüssigen Komponente, Zusammensetzung der Formulierungen

| Nr. | Polymer | flüssige Komponente | | Harz | Etofenamat |
|---|---|---|---|---|---|
| | | Polybutadien-<br>öl M.G. 1500 | Paraffin<br>dünnfl. | | |
| 03A1 | 36% | — | 45 % | 9% | 10% |
| 03B1 | 36% | 45 % | — | 9% | 10% |
| 03B2 | 36% | 22,5% | 22,5% | 9% | 10% |
| 04A1 | 36% | — | 45 % | 9% | 10% |
| 04B1 | 36% | 45 % | — | 9% | 10% |
| 04B2 | 36% | 22,5% | 22,5% | 9% | 10% |

TABELLE 6

Beispielserie C: Freisetzung in Abhängigkeit von der Zeit

| Nr. | freigesetztes Etofenamat (mg) in h | | | | | | % | Etofenamat<br>Einwaage<br>in mg |
|---|---|---|---|---|---|---|---|---|
| | 0,5 | 1 | 1,5 | 2 | 3 | 4 | | |
| Standard | 1,44 | 2,16 | 2,70 | 3,24 | 4,63 | 4,81 | 21,20 | 22,77 |
| 03A1 | 3,31 | 5,79 | 8,14 | 9,62 | 12,05 | 13,87 | 54,97 | 25,23 |
| 03B1 | 3,67 | 6,59 | 9,17 | 11,41 | 14,64 | 16,41 | 71,06 | 23,03 |
| 03B2 | 3,37 | 6,14 | 8,25 | 10,33 | 13,43 | 15,46 | 72,79 | 21,25 |
| 04A1 | 3,72 | 6,25 | 8,40 | 9,75 | 12,94 | 63,73 | 63,73 | 23,29 |
| 04B1 | 1,22 | 2,50 | 4,09 | 5,44 | 8,27 | 37,94 | 37,94 | 29,31 |
| 04B2 | 1,63 | 2,97 | 4,50 | 5,80 | 8,63 | 38,63 | 38,63 | 29,54 |

Beispielserie D:
Polymermischungen und Variationa der Wirkstoffe

Beispiel 1 (Lösung)
Styrol-Butadien-Kautschuk (Losungs—SBR)

| | |
|---|---|
| Paraffin dünnflüssig | 45,0 g |
| Polyterpenharz aus β-Pinen | 9,0 g |
| Etofenamat | 10,0 g |

Freisetzung: nach 4 Stunden 13,9 mg (54,97%)

Beispiel 2 (Lösung)

| | |
|---|---|
| Styrol-Butadien-Blockcopolymer Styrolygehalt 30%, 22% Block, $M_w$ ca. 180.000 g/mol | 45,0 g |
| Paraffin dünnflüssig | 37,5 g |
| Polyterpenharz aus β-Pinen | 9,0 g |
| Etofenamat | 10,0 g |

Freisetzung: nach 4 Std. 12,5 mg (51,74%)

Beispiel 3 (Kneter)

| | |
|---|---|
| Styrol-Butadien-Blockcopolymer, Styrolgehalt 30%, 22% Block, $M_w$ ca. 180.000 g/mol | 36,0 g |
| Polybutadienöl M.G. 1500 | 45,0 g |
| Polyterpenharz aus β-Pinen | 9,0 g |
| Etofenamat | 10,0 g |

Freisetzung: nach 4 Stunden 16,41 mg (71,06%)

Beispiel 4 (Lösung)

| | |
|---|---|
| Polybutadien-Kautschuk, 38% cis-1,4-Gehalt, 10% 1,2-Gehalt, Mooney-Viskosität 35 | 45,0 g |
| Paraffin dünnflüssig | 37,5 g |
| Polyterpenharz aus β-Pinen | 7,5 g |
| Etofenamat | 10,0 g |

Freisetzung: nach 4 Std. 12,34 mg (48,02%)

Beispiel 5 (Lösung)

| | |
|---|---|
| Polybutadien-Kautschuk, 38% cis-1,4-Gehalt, 10% 1,2-Gehalt, Mooney-Viskosität 35 | 36,0 g |
| Paraffin dünnflüssig | 45,0 g |
| Polyterpenharz aus β-Pinen | 9,0 g |
| Etofenamat | 10,0 g |

Freisetzung: nach 4 Std. 18,84 mg (63,73%)

Beispiel 6 (Lösung)

| | |
|---|---|
| Polyisobutylen M.G. 400.000 | 22,5 g |
| Polybutadien-Kautschuk, 38% cis-1,4-Gehalt 10% 1,2-Gehalt, Mooney-Viskosität 35 | 22,5 g |
| Paraffin dünnflüssig | 37,5 g |
| modifizierter Glycerinester des Colophoniums | 7,5 g |
| Etofenamat | 10,0 g |

Freisetzung: nach 4 Std. 12,45 mg (48,7%)

**Patentansprüche**

1. Medizinische Pflaster zur transdermalen Verabreichung von Antiphlogistika, bestehend aus einer Deckschicht, einer abzeiehbaren Schutzschicht und einer de Wirkstoff, Schleppmittel und Harze enthaltenden einlagigen Reservoirschicht auf Basis von Polymeren, dadurch gekennzeichnet, daß die Reservoirschicht als Polymere Butadienkautschuke mit mindestens 30% cis-1,4-Verknüpfung und Mooney-Viskositäten von 20 bis 100 (ML 1+4 bei 100°C), die bis zu 50% statistisch oder in Blöcken Styrol eingebaut enthalten können und 1 bis 30 Gew.-% des Wirkstoffs Etofenamat enthält.

2. Medizinisches Pflaster nach Anspruch 1, dadurch gekennzeichnet, daß als Polymere Butadien-Kautschuke mit Mooney-Viskositäten von 30 bis 60 (ML +4 bei 100°C) eingesetzt werden.

3. Medizinisches Pflaster nach Anspruch 1, dadurch gekennzeichnet, daß Butadien-Kautschuke mit über 80% cis-1,4-Verknüpfung eingesetzt werden.

4. Medizinisches Pflaster nach Ansprüchen 1 bis 3, dadurch gekennzeichnet, daß die Reservoirschicht neben Etofenamat 30 bis 60 Gew.-% Polymerkomponente, 30 bis 60 Gew.-% Schleppmittel und 2 bis 40 Gew.-% Harz enthält.

5. Medizinisches Pflaster nach den Ansprüchen 1 bis 4, dadurch gekennzeichnet, daß die Reservoirschicht 2 bis 20 Gew.-% Etofenamat enthält.

6. Verfahren zur Herstellung von medizinischen Pflastern nach den Ansprüchen 1 bis 4, dadurch gekennzeichnet, daß man Polybutadien-Kautschike mit mindestens 30% cis-1,4-Verknüpfung und Mooney-Viskositäten (ML 1*4 bei 100°C), die bis zu 50% statistisch oder in Blöcken Styrol eingebaut enthalten können, 1 oder mehreren Schleppmitteln und 1 oder mehrere Harze in einem Lösungsmittel löst, dann 1 bis 30 Gew.-% Etofenamat, bezogen auf Gemisch aus Polymerkomponente, Schleppmittel und Harz, ebenfalls in einem Lösungsmittel zur Lösung bringt, diese Lösungen vereinigt, und die vereinigten Lösungen gleichmäßig auf eine, für den Wirkstoff im wesentlichen undurchlässige Folie (Deckschicht) aufträgt und zu einem Film ausziehent, die beschichtete Folie (Deckschicht) zunächst bei Raumtemperatur und anschließend bei Temperaturen bis zu 50°C trocknet und gegebenenfalls die getrocknete Folie (Deckschicht) auf der beschichteten Seite mit einer für den Wirkstoff im wesentlichen undurchlässigen abziehbaren Schutzschicht versieht.

**Revendications**

1. Emplâtre médicinal pour administration transdermique d'antiphlogistiques (anti-inflammatoires), consistant en une couche de couverture, une couche amovible de protection et une couche réservoir comportant la substance, le véhicule et des résines à base de polymères, emplâtre caractérisé en ce que la couche réservoir contient comme polymère des caoutchoucs de butadiène comportant au moins 30% de liaisons en cis-1,4 et ayant des indices consistométriques Mooney de 20 à 100 (ML 1+4 à 100°C), qui peuvent contenir jusqu'à 50% de styrène incorporés de façon statistique ou en blocs ou longues séquences, et 1 à 30% en poids de la substance active étofénamate.

2. Emplâtre médicinal selon la revendication 1, caractérisé en ce qu'on utilise comme polymères des caoutchoucs de butadiène ayant des indices consistométriques Mooney de 30 à 60 (ML+4 à 100°C).

3. Emplâtre médicinal selon la revendication 1, caractérisé en ce qu'on utilise des caoutchoucs de butadiène comportant plus 80% de liaisons en cis-1,4.

4. Emplâtre médicinal selon les revendications 1 à 3, caractérisé en ce que la couche réservoir contient, en plus de l'étofénamate, 30 à 60% en poids d'un composant polymère, 30 à 60% en poids d'un véhicule et 2 à 40% en poids de résine.

5. Emplâtre médicinal selon la revendications 1 à 4, caractérisé en ce que la couche réservoir contient 2 à 20% en poids d'étofénamate.

6. Procédé pour préparer des emplâtres médicinaux selon la revendications 1 à 4, caractérisé en ce qu'on dissout des caoutchoucs de polybutadiène contenant au moins 30% de liaisons en cis-1,4 et ayant des indices consistométriques Mooney (ML 1+4 à 100°C) de 20 à 100 qui peuvent comporter jusqu'à 50% de styrène, incorporé de manière statistique ou en blocks ou longues séquences, un ou plusieurs véhicules

et une ou plusieurs résines dans un solvant, puis l'on dissout également dans un solvant 1 à 30% en poids d'étofénamate, par rapport au mélange du composant polymère, du véhicule et de la résine, on applique uniformément les solutions combinées sur une feuille (couche de couverture) essentiellement, imperméable à la substance active et l'on étale en une pellicule, on sèche tout d'abord à la température ambiante puis à des températures allant jusqu'à 50°C la feuille revêtue (feuille de couverture) et l'on munit éventuellement la feuille séchée (couche de couverture), du côté enduit, d'une couche amovible de protection essentiellement imperméable à la substance active.

**Claims**

1. Medicinal plaster for the transdermal administration of anti-inflammatory agents, comprising a covering layer, a protective layer which can be pulled off and a one-layer reservoir layer, containing the active compound, entraining agents and resins, based on polymers, characterized in that the reservoir layer contains butadiene rubbers having at least 30% cis-1,4-linkage and Mooney viscosities of 20 to 100 (ML 1+4 at 100°C), which may contain up to 50% styrene incorporated randomly or in blocks, as polymers, and 1 to 30% by weight of the active compound etofenamate.

2. Medicinal plaster according to Claim 1, characterized in that butadiene rubbers having Mooney viscosities of 30 to 60 (ML at 1+4 at 100°C) are employed as polymers.

3. Medicinal plaster according to Claim 1, characterized in that butadiene rubbers having over 80% cis-1,4-linkage are employed.

4. Medicinal plaster according to Claims 1 to 3, characterized in that the reservoir layer contains 30 to 60% by weight of polymer components, 30 to 60% by weight of entraining agent and 2 to 40% by weight of resin in addition to the etofenamate.

5. Medicinal plaster according to Claims 1 to 4, characterized in that the reservoir layer contains 2 to 20% by weight of etofenamate.

6. Method for the production of medicinal plasters according to Claims 1 to 4, characterized in that polybutadiene rubbers having at least 30% cis-1,4-linkage and Mooney viscosities (ML 1+4 at 100°C), which may contain up to 50% styrene incorporated randomly or in blocks, 1 or more entraining agents and 1 or more resins are dissolved in a solvent, then 1 to 30% by weight of etofenamate, relative to the mixture of polymer components, entraining agent and resin, are also brought into solution in a solvent, these solutions are combined, and the combined solutions are uniformly applied to a layer (covering layer) which is essentially impermeable to the active compound and drawn out to give a film, the coated layer (covering layer) is dried first at room temperature and then at tempertures up to 50°C and, if appropriate, the dried layer (covering layer) is provided on the coated side with a protective layer which is essentially impermeable to the active compound and which can be pulled off.

EP 0 150 428 B1

FIG. 1

FIG. 2